# EUROPEAN PATENT APPLICATION

(11) **EP 4 793 276 A1**
(43) Date of publication of application: **19.08.2026**
(21) Application number: 23957027.8
(22) Date of filing: 30.10.2023
(51) Int. Cl.: C07D 471/04, A61K 51/04, A61K 31/437, A61P 13/12

(54) **DRUG FOR DIAGNOSING DIABETIC NEPHROPATHY AND USE THEREOF**

(71) Applicant: Fudan University, Shanghai 200433 (CN); Shanghai Chartwell Medical Science & Technology Co., Ltd., Shanghai 201206 (CN)
(72) Inventor: JI, Bin, Shanghai 200433 (CN); KAI, Mototora, Shanghai 201206 (CN)
(74) Representative: Barker Brettell LLP
(86) International application number: PCT/CN2023/127509
(87) International publication number: WO 2025/091145

(57) **Abstract**

The present invention provides a compound represented by general formula (I) or a pharmaceutically acceptable salt, precursor, or solvate thereof, and a use thereof in the preparation of a drug for diagnosing diabetic nephropathy. 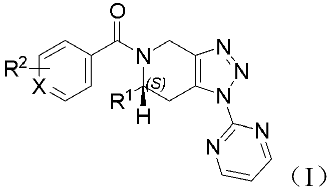 wherein X is selected from carbon or nitrogen; R¹ is selected from hydrogen or alkyl; R² is one or more substituents on the benzene ring or the pyridine ring, and R² is independently selected from one or more of hydrogen, halogen, hydroxyl, cyano, nitro, amino, C1-C6 alkyl, C3-C6 cycloalkyl, C2-C6 alkenyl, C2-C6 alkynyl, C1-C6 alkoxy, C3-C6 cycloalkoxy, C1-C6 alkylamino, C3-C6 cycloalkylamino, halo C1-C6 alkyl, halo C3-C6 cycloalkyl, halo C1-C6 alkoxy, halo C3-C6 cycloalkoxy, halo C1-C6 alkylamino, halo C3-C6 cycloalkylamino, C6-C8 aryl, or C5-C8 heteroaryl, and in these substituents represented by R², at least one atom or the entire substituent is replaced by a radionuclide. The drug and method for diagnosing diabetic nephropathy according to the present invention relate to non-invasive diagnosis technology and have the advantages of causing less pain to a subject and having less contraindications.

## Description

### TECHNICAL FIELD

This invention relates to diagnostic drugs for diabetic nephropathy, particularly radiotherapy drugs, and specifically, to the use of a triazolotetrahydropyridine cyclic compound in the diagnosis of diabetic nephropathy and a diagnostic method for diabetic nephropathy.

### BACKGROUND TECHNOLOGY

Diabetic nephropathy (DKD) is a common complication of diabetes and one of the important causes of end-stage renal failure. Early diagnosis of DKD is particularly important. Studies generally suggest that kidney function damage is irreversible when the estimated glomerular filtration rate (eGFR) is <30 mL/min⁻¹·1.73 mL/min⁻². Early drug intervention can delay the onset of organic kidney disease. Early detection of glomerular, tubular, and interstitial lesions is beneficial for the early diagnosis and treatment of DKD. A retrospective study involving 121,395 participants showed that early diagnosis of DKD could reduce the risk of progression to end-stage renal disease by 80% (Chinese Journal of Internal Medicine. 2021. 60(6): 522-532.). Because the management and treatment of diabetic kidney disease (DKD) differ significantly from those of non-diabetic kidney disease (NDKD), clinicians should accurately diagnose DKD and identify NDKD based on clinical manifestations and auxiliary examinations, so as to provide timely and appropriate treatment to the patients. Currently, the diagnosis of DKD in clinical practice is often based on albuminuria and whether the subject has diabetic retinopathy. However, albuminuria is not specific for diagnosing DKD, and some DKD subjects may show negative urine protein in the early stages, with only a decrease in GFR. Therefore, the presence of albuminuria or a decrease in GFR in diabetic subjects can indicate DKD, NDKD, or a combination of both. Therefore, before diagnosing DKD, it is necessary to first rule out whether diabetes is complicated with NDKD. Currently, renal biopsy is the gold standard for diagnosing DKD and ruling out NDKD. If the subject has no contraindications for renal biopsy, renal biopsy pathological diagnosis of DKD is recommended (Expert Consensus on the Prevention and Treatment of Diabetic Nephropathy, 2014 edition).

Japanese Patent Application Publication No. 2010-256132A discloses a method for detecting the progression of diabetic nephropathy, a diagnostic kit for the progression of diabetic nephropathy, a substance as an indicator of the progression of diabetic nephropathy. In this patent application, bodily fluids collected from the subject are reacted with lectins, the amount of glycans with affinity for lectins is measured, and the measured amount of glycans is used to detect the progression of diabetic nephropathy.

International patent application WO2020071517 discloses a biomarker miRNA-125b-5p and/or miRNA-181b-5p that can specifically diagnose diabetic nephropathy. In this diagnosis, diabetic nephropathy is diagnosed when the expression of miRNA-125b-5p in the blood is increased and/or the expression of miRNA-181b-5p is decreased.

### SUMMARY OF THE INVENTION

The technical problem to be solved by this invention

As mentioned above, the most effective method for diagnosing diabetic nephropathy is renal biopsy. However, renal biopsy is an invasive procedure, and renal biopsy must excludes contraindication such as significant bleeding tendency, severe hypertension, mental illness or non-cooperative patients, solitary kidney, and small kidney. Therefore, kidney biopsy has certain limitations.

In view of the limitations of the prior art, the purpose of this invention is to provide a non-invasive and contraindication-free diagnostic drug and method for diabetic nephropathy.

Technical solutions to technical problems

The inventors sought compounds that specifically accumulate in the kidneys of subjects with diabetic nephropathy. Among the compounds described in International Publication No. WO2014/152604A1, which discloses affinity for the P2X7 receptor (P2X7R), the inventors found that compounds shown by the following general formula (I) have the property of specifically accumulating in the kidneys of subjects/models with diabetic nephropathy. Radionuclide labeling to the aforementioned compounds and positron emission tomography (PET) or single photon emission computed tomography (SPECT) imaging techniques are used in this invention to locate and quantify the aforementioned compounds accumulated in the kidney, thereby achieving the purpose of diagnosing diabetic nephropathy.

Specifically, the present invention includes the following technical solutions.

A compound shown by general formula (I) or pharmaceutically acceptable salt, precursor, and solvate thereof, 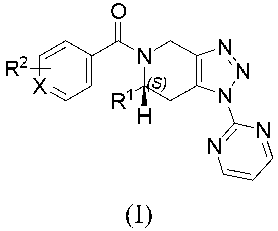 wherein, X is selected from carbon(C) or nitrogen(N);
R¹ is selected from hydrogen or alkyl;
R² is one or more substituents on a benzene ring or pyridine ring, and R² is independently selected from one or more of hydrogen, halogen, hydroxyl, cyano, nitro, amino, C1-C6 alkyl, C3-C6 cycloalkyl, C2-C6 alkenyl, C2-C6 alkynyl, C1-C6 alkoxy, C3-C6 cycloalkoxy, C1-C6 alkylamino, C3-C6 cycloalkylamino, C1-C6 hydroxyalkyl, C3-C6 hydroxycycloalkyl, halo C1-C6 alkyl, halo C3-C6 cycloalkyl, halo C1-C6 alkoxy, halo C3-C6 cycloalkoxy, halo C1-C6 alkylamino, halo C3-C6 cycloalkylamino, C6-C8 aryl or C5-C8 heteroaryl, and in these substituents represented by R², at least one atom or the entire substituent is replaced by a radionuclide.

In the compound shown by general formula (I), R¹ is preferably hydrogen, C1-C6 alkyl, or C3-C6 cycloalkyl.

In the compound shown by general formula (I), R² is one or more substituents on the benzene ring or pyridine ring, for example, there may be 1, 2, 3, 4 or 5 substituents. When there are 2 substituents or more, these substituents may be the same or different.

R² is further preferably one or more of hydrogen, halogen, hydroxyl, nitro, amino, C1-C4 alkyl, C1-C4 alkoxy, C1-C4 alkylamino, halo C1-C4 alkyl, halo C1-C4 alkoxy or halo C1-C4 alkylamino, and at least one atom of the substituent or the entire substituent is replaced by a radionuclide.

In the compound shown by general formula (I), the radionuclide is preferably one or more of ¹⁸F, ¹¹C, ¹³¹I, ¹²³I, ¹²⁴I, or ¹²⁵I.

The compound shown by general formula (I) is preferably selected from any one of the following compounds: 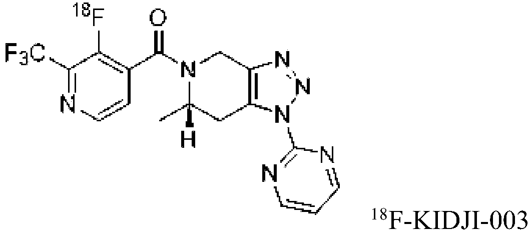
(S)-(3-fluoro-2-trifluoromethylpyridin-4-yl)(6-methyl-1-(pyrimidin-2-yl)-1,4,6,7-tetrahydro-5H-[1,2,3]triazolo[4,5-c]pyridin-5-yl) methyl ketone, 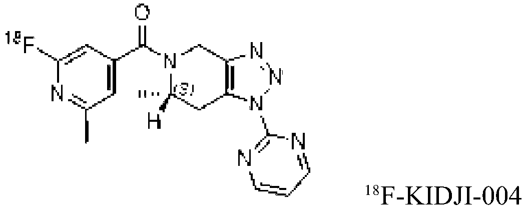
(S)-(2-fluoro-6-methylpyridin-4-yl)(6-methyl-1-(pyrimidin-2-yl)-1,4,6,7-tetrahydro-5H-[1,2,3]triazolo[4,5-c]pyridin-5-yl) methyl ketone, 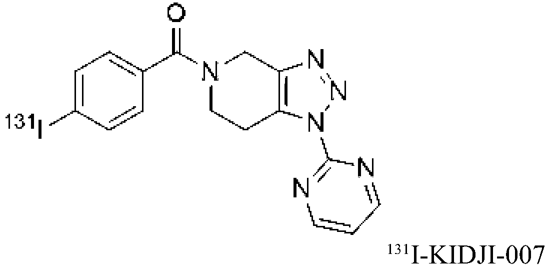
(4-iodophenyl)(1-(pyrimidin-2-yl)-1,4,6,7-tetrahydro-5H-[1,2,3]triazolo[4,5-c]pyridin-5-yl) methyl ketone, 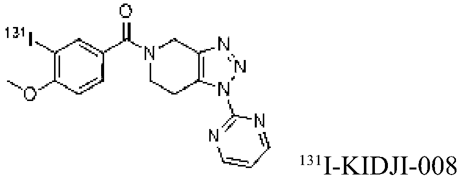
(3-iodo-4-methoxyphenyl)(1-(pyrimidin-2-yl)-1,4,6,7-tetrahydro-5H-[1,2,3]triazolo[4,5-c]pyridin-5-yl) methyl ketone, 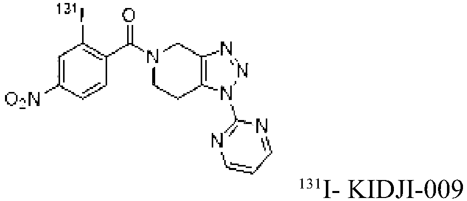
(2-iodo-4-nitro)(1-(pyrimidin-2-yl)-1,4,6,7-tetrahydro-5*H*-[1,2,3]triazolo[4,5-c]pyridin-5-yl) methyl ketone,
(2-Fluoro-4-(tri-n-butyltin)phenyl)(1-(pyrimidin-2-yl)-1,4,6,7-tetrahydro-5H-[1,2,3]triazolo[4,5-c]pyridin-5-yl) methyl ketone, 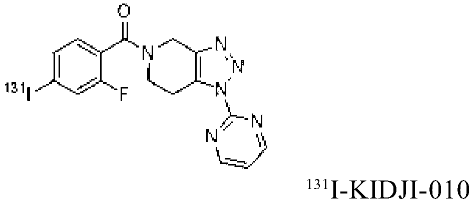 (2-fluoro-4-iodophenyl)(1-(pyrimidin-2-yl)-1,4,6,7-tetrahydro-5H-[1,2,3]triazolo[4,5-c]pyridin-5-yl) methyl ketone.

The precursor compound is selected from: 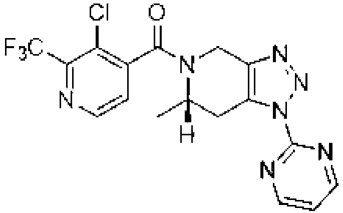
precursor of KIDJI-003 (S)-(3-chloro-2-trifluoromethylpyridin-4-yl)(6-methyl-1-(pyrimidin-2-yl)-1,4,6,7-tetrahydro-5H-[1,2,3]triazolo[4,5-c]pyridin-5-yl) methyl ketone, 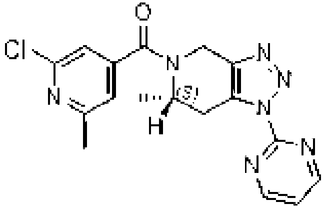
precursor of KIDJI-004 (S)-(2-chloro-6-methylpyridin-4-yl)(6-methyl-1-(pyrimidin-2-yl)-1,4,6,7-tetrahydro-5*H*-[1,2,3]triazolo[4,5-c]pyridin-5-yl) methyl ketone, 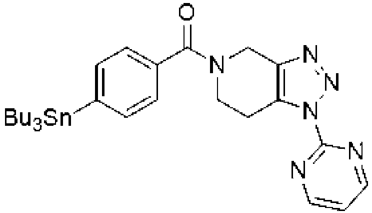
precursor of KIDJI-007 (1-(pyrimidin-2-yl)-1,4,6,7-tetrahydro-5*H*-[1,2,3]triazolo[4,5-c]pyridin-5-yl)(4-tri-n-butyltinphenyl) methyl ketone, 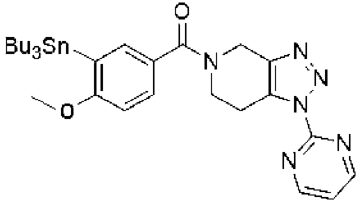
precursor of KIDJI-008 (4-methoxy-3-(tri-n-butyltin)phenyl)(1-(pyrimidin-2-yl)-1,4,6,7-tetrahydro-5H-[1,2,3]triazolo[4,5-c]pyridin-5-yl) methyl ketone, 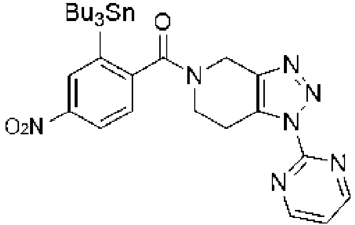
precursor of KIDJI-009 (4-nitro-2-(tri-n-butyltin)phenyl)(1-(pyrimidin-2-yl)-1,4,6,7-tetrahydro-5*H-*[1,2,3]triazolo[4,5-c]pyridin-5-yl) methyl ketone, 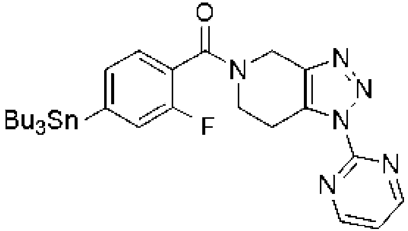
precursor of KIDJI-010 (2-fluoro-4-(tri-n-butyltin)phenyl)(1-(pyrimidin-2-yl)-1,4,6,7-tetrahydro-5*H-*[1,2,3]triazolo[4,5-c]pyridin-5-yl) methyl ketone.

The pharmaceutically acceptable salt of the compound of the present invention are addition salt of inorganic acid or organic acid. The inorganic acid is selected from hydrochloric acid, hydrobromic acid, hydroiodic acid, sulfuric acid, phosphoric acid and nitric acid, and the organic acid is selected from acetic acid, tartaric acid, salicylic acid, methanesulfonic acid, succinic acid, citric acid, malic acid, lactic acid and fumaric acid.

The solvate of the compound of the present invention is preferably a hydrate.

The present invention also provides use of any of the above-mentioned compound or its pharmaceutically acceptable salt, precursor, or solvate in the preparation of diagnostic drugs for diabetic nephropathy.

The present invention also provides a radiodiagnostic kit for diagnosing diabetic nephropathy, the radiodiagnostic kit comprises a compound having the structure shown in general formula (I) or a pharmaceutically acceptable salt, precursor, or solvation thereof.

In the diagnostic kit of the present invention, the pharmaceutically acceptable salt is an addition salt of an inorganic acid or an organic acid. The inorganic acid is selected from hydrochloric acid, hydrobromic acid, hydroiodic acid, sulfuric acid, or phosphoric acid, and the organic acid is selected from acetic acid, tartaric acid, salicylic acid, methanesulfonic acid, succinic acid, citric acid, malic acid, lactic acid and fumaric acid.

The solvate of the compound of the present invention is preferably a hydrate.

The present invention also provides a method for diagnosing diabetic nephropathy, comprising:
prepare physiologically acceptable solutions of any of the compound described above, or its pharmaceutically acceptable salt, precursor, or solvate,
introduce the solution into the subject's body,
form an image for the subject's kidneys,
analyze the image, and make a diagnosis based on the analysis results.

In the diagnostic method described above, form an image via PET or SPECT is preferred.

In the diagnostic method described above, the physiologically acceptable solution can be introduced into the subject's body, for example, by injection.

### Beneficial technical effects

This invention provides a radioactive diagnostic drug, its application in the diagnosis of diabetic nephropathy, and a method for diagnosing diabetic nephropathy. By administering the aforementioned radiodiagnostic drug intravenously followed by PET or SPECT imaging, non-invasive diagnosis of diabetic nephropathy can be achieved. Compared with existing invasive kidney biopsies, the present invention has the advantages of less pain for subjects and fewer contraindications.

### BRIEF DESCRIPTION OF THE DRAWINGS

FIG.1 shows ex vivo autoradiography images of ¹⁸F-KIDJI-003 in normal mice and diabetic nephropathy model mice.
FIG.2 shows the quantitative analysis results of ex vivo autoradiography of ¹⁸F-KIDJI-003 in normal mice and diabetic nephropathy model mice.
FIG.3 shows SPECT in vivo images of the kidneys of the radiolabeled compounds ¹⁸F-KIDJI-004, ¹³¹I-KIDJI-007, ¹³¹I-KIDJI-008, ¹³¹I-KIDJI-009, and ¹³¹I-KIDJI-010 of the present invention in db/db model mice and normal mice.
FIG.4 shows in vitro autoradiography images of ¹⁸F-KIDJI-003 from the kidneys of normal mice, diabetic nephropathy mice (heterozygous and homozygous).
FIG.5 shows the immunostaining results of P2X7R in the kidneys of normal mice, diabetic nephropathy mice (heterozygous and homozygous).

### DETAILED DESCRIPTION OF THE EMBODIMENTS

The features, properties, compounds, chemical parts or groups described in connection with embodiments or examples of the present invention should be understood to be applicable to any other embodiment or examples described herein, unless incompatible therewith. All features disclosed in this specification (including the claims, abstract, and drawings) and/or all steps of any method or process disclosed therefrom may be combined in any way, except for at least some mutually exclusive combinations of these features and/or steps. This invention is not limited to the details of any particular embodiment. This invention extends to any new feature or any new combination of features disclosed in this specification (including the claims, abstract, and drawings), or to any new step or any new combination of steps in any method or process disclosed herein.

### Imaging isotopes and imaging:

Diagnostic techniques in nuclear medicine use radioactive tracers that emit γ rays from within the body. These tracers are typically short-lived isotopes conjugated to compounds, which allow for detailed examination of specific physiological processes. They can be administered by injection, inhalation, or oral administration. The first method involves using a gamma camera to detect individual photons, which can observe organs from many different angles. The camera creates an image based on the point of radiation emission, this image is enhanced by a computer, and doctors view it on a monitor for signs of abnormality.

Positron emission tomography (PET) is a precise and sophisticated technique that uses isotopes produced by a cyclotron. Positron-emitting radionuclide is typically introduced via injection and accumulates in target tissue. When the radionuclide decays, it emits a positron, which rapidly combines with nearby electrons, resulting in the simultaneous emission of two identifiable gamma rays in opposite directions. These are detected by PET cameras and their origins are indicated with great precision. The most important clinical application of PET is in oncology, using fluoro-18-fluorodeoxyglucose ([¹⁸F]FDG) as a tracer, as it has been proved to be the most accurate non-invasive method for detecting and assessing most cancers. It is also well used for cardiac and brain imaging.

Many medical diagnostic procedures, including PET and SPECT, utilize radiolabeled compounds, as is well known in the field. PET and SPECT are highly sensitive techniques and require small amounts of radiolabeled compounds (called tracers). Radiolabeled compounds are transported, accumulated, and transformed in the body in a similar manner to their corresponding non-radiolabeled compounds. The tracer or probe can be radiolabeled using radionuclides useful for PET imaging, such as ¹¹C, ¹³N, ¹⁵O, ¹⁸F, ⁶⁴Cu and ¹²⁴I, or using radionuclides useful for SPECT imaging, such as ⁹⁹Tc, ⁷⁷Br, ⁶¹Cu, ¹⁵³Gd, ¹²³I, ¹²⁵I, ¹³¹I and ³²P. These are nonlimiting examples of the term " radionuclide" (also known as radioisotope, imaging isotope) used in this description.

Regarding radioactive halogens, the isotope ¹²³I has a half-life of 13 hours and a gamma energy of 159 keV, so ¹²³I or ¹⁸F (half-life of 2 hours) is usually used to label ligands for diagnostic purposes. Other imaging isotopes that can be used include ¹³¹I, ⁷⁷Br, and ⁷⁶Br.

Those skilled in the art are familiar with various ways of detecting labeled compounds for imaging purposes. For example, positron emission tomography (PET) or single-photon emission computed tomography (SPECT) can be used to detect radiolabeled compounds. The labeling introduced into a compound may depend on the required detection method. Those skilled in the art are familiar with the PET detection of positron-emitting atoms such as ¹⁸F. Those skilled in the art are familiar with SPECT detection of photon-emitting atoms such as ¹²³I or ⁹⁹Tc.

Radioactive diagnostic or detection reagents should have sufficiently high radioactivity and radioactive concentration to ensure reliable diagnosis and detection. The required level of radioactivity can be obtained using the methods described herein for preparing the compound.

Typically, in the first step of an imaging method, a labeled compound is introduced into the tissue or the subject in a detectable amount. This compound is typically part of a pharmaceutical composition and is administered to a tissue or subject using methods well known to those skilled in the art. Normally, it is administered via intravenous injection.

In other embodiments of this invention, the labeled compound is introduced into a subject in a detectable amount, and after sufficient time has been allowed for the compound to accumulate in the kidneys, the labeled compound is detected noninvasively.

The detectable amount is the amount of labeled compound necessary for the selected detection method to detect it. The amount of the labeled compound to be introduced into a subject to provide a detectable amount can be easily determined by those skilled in the art. For example, the amount of the labeled compound given to the subject can be gradually increased until the compound is detected using the selected detection method. Introducing radionuclides into compound to enable the detection of this compound.

The required time amount can be easily determined by introducing a detectable amount of the labeled compound into the subject and then detecting the labeled compound at different times after administration.

Radiolabeled compound may be administered to a subject by means of an overall or local administration route. For example, a labeled compound can be administered to a subject, thereby delivering the compound throughout the body. Alternatively, the labeled compound may be applied to a specific target organ or tissue. For example, it may be desirable to locate and quantify the levels of labeled compounds in the kidneys in order to diagnose or track the progression of diabetic nephropathy in a subject.

By replacing one or more atoms (e.g., hydrogen, alkyl, or halogen, etc.) of compound with affinity for the P2X7 receptor (P2X7R) described in the prior art WO2014/152604A1 with a radionuclide, it is possible that one or more radionuclides are incorporated into the compounds disclosed in the present invention. The incorporation of radionuclides can be carried out using known techniques. For example, these techniques can be based on nucleophilic or electrophilic ¹⁸F fluorination of suitable precursors, such as those reviewed in the following literature: Medicinal Chemistry Approaches to Personalized Medicine (Lackey, Roth, ed.), Chapter 12 (Wiley-VCH, ISBN 978-3-527-33394-3).

### DEFINITION

In the examples of the present invention, unless otherwise specified, all instruments, raw material components, experimental animals, and chemical reagents are commercially available products well known to those skilled in the art. In the examples of the present invention, unless otherwise specified, the technical means used are conventional means well known to those skilled in the art. The progress of the reaction in this invention can be monitored using conventional monitoring methods in the art (e.g., TLC, HPLC, LC-MS or NMR), and the reaction endpoint is generally defined as the disappearance of the reaction substrate.

Experimental methods for which specific conditions are not specified in the examples of the specification are generally performed under conventional conditions in the art or under conditions recommended by the manufacturer. In this invention, unless otherwise specified, "and/or above", "and/or below", and "within" indicate that the number itself is included. When this description uses "comprising" or "including" to describe embodiments, other similar embodiments described as "consisting of ..." and/or "substantially consisting of ..." are also provided. When this description describes an embodiment as "substantially consisting of...", other similar embodiment described as "consisting of..." are also provided. The term "and/or" as used in phrases such as "A and/or B" in this description is intended to include both A and B, A or B, A (alone), and B (alone). Similarly, the term "and/or" used in phrases such as "A, B and/or C" is intended to include each of the following embodiments: A, B and C; A, B or C; A or C; A or B; B or C; A and C; A and B; B and C; A (alone); B (alone); and C (alone).

In the present invention, the term "C1-C6 alkyl" refers to a straight-chain or branched alkyl group having 1 to 6 carbon atoms in its chain. For example, methyl (Me), ethyl (Et), n-propyl, isopropyl, n-butyl, isobutyl, tert-butyl (tBu), n-pentyl, isopentyl (2-methylbutyl), neopentyl (2,2-dimethylpropyl), n-hexyl, 2-methylpentyl, 3-methylpentyl, 2,3-dimethylbutyl, 2,4-dimethylbutyl, 3,3-dimethylbutyl, 2-ethylbutyl, etc., and groups that, based on ordinary skill in the art and the teachings provided herein, would be considered equivalent to any of the foregoing examples. Similarly, the term "C1-C4 alkyl" refers to straight-chain or branched alkyl groups having 1 to 4 carbon atoms in the chain.

The term "C3-C6 cycloalkyl" refers to a saturated monocyclic or polycyclic carbon ring having 3 to 6 carbon atoms, such as cyclopropyl, 1-methylcyclopropyl, cyclobutyl, cyclopentyl, 1-methylcyclobutyl, cyclohexyl, 1,3-dimethylcyclobutyl, 1-methylcyclopentyl, and any group that, based on ordinary skill in the art and the teachings provided herein, would be considered equivalent to any of the foregoing examples.

The term "C2-C6 alkenyl" refers to a chain with 2 to 6 carbon atoms and containing at least one carbon-carbon double bond, which can be straight or branched. The group may contain multiple double bonds, and the orientation of each double bond is independently E or Z. The alkenyl group is preferably 1-alkenyl. Exemplary alkenyl groups include, but are not limited to, vinyl, propenyl, butenyl, pentenyl, hexenyl, and groups that, based on ordinary skill in the art and the teachings provided herein, would be considered equivalent to any of the foregoing examples. The group can be a terminal group or a bridging group.

The term "C2-C6 alkynyl" refers to a group having 2 to 6 carbon atoms and containing at least one carbon-carbon triple bond, which can be straight-chain or branched. The group may contain multiple triple bonds.The alkynyl group is preferably 1-alkynyl. Exemplary alkynyl groups include, but are not limited to, ethynyl, propynyl, butynyl, pentynyl, hexynyl, and groups that, based on ordinary skill in the art and the teachings provided herein, would be considered equivalent to any of the foregoing examples. The group can be a terminal group or a bridging group.

The term "C1-C6 alkoxy" refers to a C1-C6 alkyl-O-group, wherein the C1-C6 alkyl group is as defined above in this description. The group can be a terminal group or a bridging group. Specifically, C1-C6 alkoxy groups include methoxy, ethoxy, propoxy, isopropoxy, butoxy, isobutoxy, sec-butoxy, tert-butoxy, n-pentoxy, isopentoxy (e.g., 2-methylbutoxy, 2-methoxybutyl), neopentoxy (e.g., 2,2-dimethylpropoxy), cyclopentoxy, n-hexyloxy, 2-methylpentoxy, 2-methoxypentyl, 3-methylpentoxy, 3-methoxypentyl, 2,3-dimethylbutoxy, 2,4-dimethylbutoxy, 3,3-dimethylbutoxy, 2,3-dimethoxybutyl, 2,4-dimethoxybutyl, 3,3-dimethoxybutyl, 2-ethylbutoxy, 2-ethoxybutyl, etc., and groups that, based on ordinary skill in the art and the teachings provided herein, would be considered equivalent to any of the foregoing examples.

The term "C1-C6 alkylamino" refers to an NH₂-alkyl (C1-C6) group, wherein definitions of C1-C6 alkyl groups are the same as above. The group can be a terminal group or a bridging group. If the group is a terminal group, then the group is bonded to the rest of the molecule via an alkyl group.

The term "halogen" refers to fluorine, chlorine, bromine, or iodine.

The term "halo C1-C6 alkyl" refers to C1-C6 alkyl groups in which hydrogen is optionally replaced by a halogen. The definitions of C1-C6 alkyl groups are the same as above. Examples include, but are not limited to, trifluoromethyl (CF₃), difluoromethyl (CF₂H), monofluoromethyl (CH₂F), pentafluoroethyl (CF₂CF₃), tetrafluoroethyl (CHFCF₃), monofluoroethyl (CH₂CH₂F), trifluoroethyl (CH₂CF₃), tetrafluorotrifluoromethylethyl (-CF(CF₃)₂), and groups that, based on ordinary skill in the art and the teachings provided herein, would be considered equivalent to any of the foregoing examples.

The term "halo C1-C6 alkoxy" refers to a C1-C6 alkoxy group in which hydrogen is optionally replaced by a halogen. The definitions of C1-C6 alkoxy groups are the same as above. Examples of haloalkoxy groups include trifluoromethoxy (OCF₃), difluoromethoxy (OCF₂H), monofluoromethoxy (OCH₂F), monofluoroethoxy (OCH₂CH₂F), pentafluoroethoxy (OCF₂CF₃), tetrafluoroethoxy (OCHFCF₃), trifluoroethoxy (OCH₂CF₃), tetrafluorotrifluoromethylethoxy (-OCF(CF₃)₂), and groups that, based on ordinary skill in the art and the teachings provided herein, would be considered equivalent to any of the foregoing examples.

The term "halo C1-C6 alkylamino" refers to a C1-C6 alkylamino group in which hydrogen is optionally replaced by a halogen. The definitions of C1-C6 alkylamino groups are the same as above.

The term "C6-C8 aryl" refers to an aromatic carbon ring having a ring structure in which all the ring atoms are carbon, preferably having 6 to 8 carbon atoms per ring. Examples of aryl include phenyl. The group can be a terminal group or a bridging group.

The term "C5-C8 heteroaryl" refers to a group containing an aromatic ring (preferably a 5- or 6-membered aromatic ring) having one or more heteroatoms as ring atoms in the aromatic ring, while the remaining ring atoms are carbon atoms. Suitable heteroatoms include nitrogen, oxygen, and sulfur. Examples of heteroaryl groups include thiophene, furan, imidazole, thiazole, isothiazole, [2,3-b]thiophene, isoindazine, pyrrole, pyrazole, pyridine, pyrazine, pyrimidine, pyridazine, tetrazolium, indole, isoindole, 1H-indazole, purine, quinoline, isoquinoline, phthalazine, naphthidine, quinoxaline, cyclophosphine, carbazole, phenanthridine, acridine, phenazine, isothiazole, phenothiazine, oxazole, isoxazole, furazine, phenothiazine, 2-pyridyl, 3-pyridyl or 4-pyridyl, 2-quinolinyl, 3-quinolinyl, 4-quinolinyl, 5-quinolinyl or 8-quinolinyl, 1-isoquinolinyl, 3-isoquinolinyl, 4-isoquinolinyl or 5-isoquinolinyl, 1-indole, 2-indole or 3-indole, and 2-thienyl or 3-thienyl. The group can be a terminal group or a bridging group.

The term "substitute" or "replace" refers to a specified group or part having one or more substituent. The term "optionally substituted" means that a particular group is either unsubstituted or substituted by one or more substituent. If the term "substitution" is used to describe a structural system, it means that substitution occurs at any position in the system where the valence is allowed. Where a specified portion or group is not explicitly indicated to be optionally substituted or replaced by any specified substituent, it should be understood that such portion or group is intended to indicate that it is unsubstituted. The terms "one or more" used in the context of substituent refer to the range from a single substituent to the highest chemically possible number of substituents, i.e., replacing one atom with its corresponding radioactive isotope, until all atoms are substituted.

The term "diagnosis" refers to the act of identifying a disease from its signs and symptoms. In this invention, it specifically refers to the analysis of biomarkers that indicate a disease.

The term "subject" includes both humans and non-human animals.Non-human animals include all vertebrates, such as mammals and non-mammals, such as non-human primates, sheep, dogs, cattle, chickens, amphibians, and reptiles. Unless otherwise specified, the terms "patient" or "subject" are used interchangeably in this description.

The term "P2X7R" refers to the P2X7 receptor.

The term "pharmaceutically acceptable" refers to a product or compound approved by the Chinese drug regulatory authority or listed in the Chinese Pharmacopoeia or other recognized pharmacopoeias for use in animals, including humans.

The "precursor" described in the present invention refers to a chemical substance at the stage preceding the formation of the compound of general formula (I). This chemical substance can be converted into a radiolabeled compound of formula (I) through a one-step radiolabeling chemical reaction.

Compound of general formula (I) can form addition salt with suitable nontoxic organic or inorganic acid. Examples of acid addition salt include salt derived from inorganic acid such as hydrochloric acid, hydrobromic acid, hydroiodic acid, sulfuric acid, phosphoric acid, and nitric acid, and salt derived from organic acid such as acetic acid, tartaric acid, salicylic acid, methanesulfonic acid, succinic acid, citric acid, malic acid, lactic acid, or fumaric acid, etc. These salts can be prepared from compounds of general formula (I) according to known salt-forming methods.

The compounds of the present invention can be recovered from the reaction mixture and purified in a conventional manner. Isomers, such as enantiomers, can be obtained in a conventional manner, for example, by stepwise synthesis from the corresponding asymmetrically substituted starting materials. If necessary, the protection of any active group can be carried out in any suitable step. The suitable protecting base is a protecting base that is conventionally used in the prior art, and can be introduced and removed using conventional methods. For example, when an amino group is protected by Boc, the Boc can be removed under acidic conditions using conventional methods.

The compounds of the present invention can be synthesized according to prior arts. The present invention will be described in detail below with reference to the examples.

### Example 1: Method for preparing the precursor compound of the present invention

The precursor compound of the present invention was prepared according to the following flowchart.

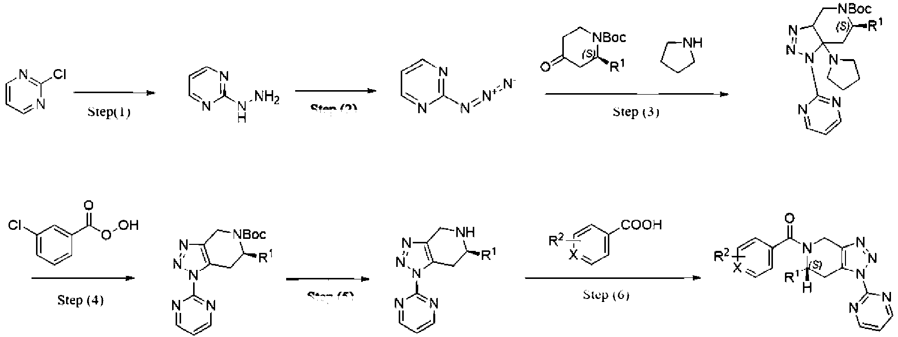

### Example 1-1:

### Synthesis of (S)-(3-chloro-2-(trifluoromethyl)pyridin-4-yl)(6-methyl-1-(pyrimidin-2-yl)-1,4,6,7-tetrahydro-5H-[1,2,3]triazolo[4,5-c]pyridin-5-yl) methyl ketone (precursor of KIDJI-003)

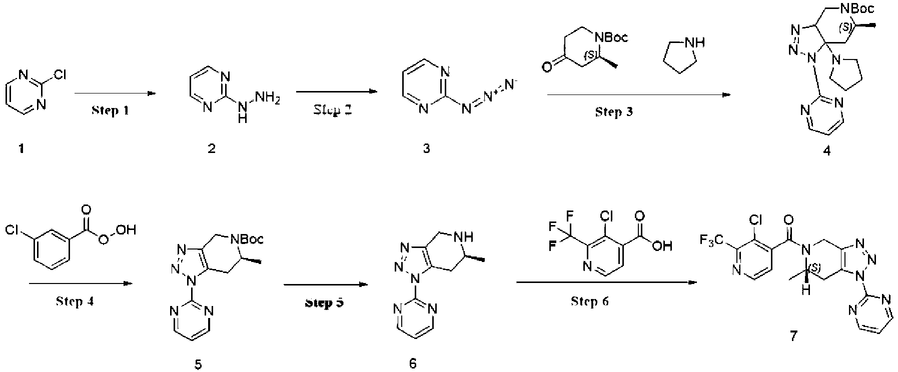

### Step 1. Synthesis of Compound 2

2-Chloropyrimidine (10.0 g, 1.0 eq) was placed in a 100 mL three-necked flask under N₂ gas protection and the temperature was controlled at T≤30°C. Hydrazine hydrate (20 mL, 4.7 eq) was slowly added dropwise and stirred overnight at room temperature for 16 h. The reaction was monitored by TLC until the starting material was completely reacted(pure ethyl acetate, product polarity increased). Concentrate under reduced pressure to dryness, add n-hexane (50 mL) and slurry for 2 hours, filter, wash the filter cake with n-hexane (20 mL), concentrate the solid to dryness, and pump dry to obtain 7.7 g of white solid, yield 80%.

### Step 2. Synthesis of Compound 3

Compound 2 (5 g, 1.0 eq) was placed in a 250 mL three-necked flask under N₂ gas protection. 100 mL of 50% glacial acetic acid aqueous solution was added, and the temperature was lowered to 0 °C or below. A 25 mL aqueous solution of sodium nitrite (6.3 g, 2.0 eq) was slowly added dropwise, with the temperature controlled at T ≤ 5 °C. The addition was completed in half an hour. The reaction was carried out at 0 °C for about 2 hours. After the reaction was completed(monitored by TLC), sodium carbonate solid was slowly added to adjust the pH to about 8. The mixture was extracted three times with toluene, and the organic layer was collected, dried, filtered, and used directly for the next reaction step without concentration.

### Step 3. Synthesis of Compound 4

Compound 3 (0.7 g, 1.0 eq) and (S)-2-methyl-4-oxopiperidin-1-carboxylic acid tert-butyl ester (0.95 g, 0.77 eq) were placed in a 100 mL three-necked flask, toluene (10 mL) was added, and the mixture was protected with N₂. The temperature was raised to 70 °C, and tetrahydropyrrole (0.316 g, 0.77 mmol) was injected using a syringe. The temperature was raised to 100 °C, and the reaction was allowed to proceed for 16 h. TLC monitoring showed that new spots were formed.Cool to room temperature, concentrate under reduced pressure, and purify by column chromatography (PET/EA=1:10).

### Step 4.Synthesis of Compound 5

Compound 4 (0.5 g, 1.0 eq) was placed in a 100 mL three-necked flask under N₂ protection. Anhydrous dichloromethane (10 mL) and sodium bicarbonate (0.11 g, 1.0 eq) were added. Then, m-CPBA (0.33 g, 1.0 eq) was slowly added. The mixture was stirred at room temperature for 2 hours, and the reaction was monitored by TLC until the starting material was completely reacted. After the reaction was complete, 10 mL of 1N sodium hydroxide aqueous solution was added, and the mixture was stirred for 20 minutes. The mixture was then extracted with dichloromethane, the organic phase was collected, dried, concentrated under reduced pressure, and used directly in the next reaction step.

### Step 5. Synthesis of Compound 6

Compound 5 (1 g, 1.0 eq) was placed in a 100 mL single-necked flask under N₂ protection. TFA (2.4 g, 10 eq) was added, and the mixture was stirred at room temperature for 16 h. The reaction was monitored by TLC until the starting material was completely reacted. The mixture was concentrated under reduced pressure to remove TFA, and dichloromethane was added. The pH was adjusted to approximately 8 by adding 10% sodium carbonate aqueous solution. The mixture was extracted three times with dichloromethane, and the organic phase was collected, dried, filtered, concentrated under reduced pressure, and purified by column chromatography (DCM/MeOH=15:1).

### Step 6. Synthesis of (S)-(3-chloro-2-(trifluoromethyl)pyridin-4-yl)(6-methyl-1-(pyrimidin-2-yl)-1,4,6,7-tetrahydro-5H-[1,2,3]triazolo[4,5-c]pyridin-5-yl) methyl ketone (compound 7; precursor of KIDJI-003)

Compound 6 (60 g, 1.0 eq) was placed in a 100 mL three-necked flask under N₂ protection, and chloronicotinic acid (50 mg, 1.1 eq), HATU (91 mg, 1.1 eq), DIPEA (0.15 mL, 4.0 eq), DMF (5 mL), and DCM (10 mL) were added. The mixture was stirred at room temperature for 16 h. LCMS monitored the formation of a product. Wash with 20 ml of saturated saline solution, extract with dichloromethane, collect the organic phase, concentrate under reduced pressure, purify by column chromatography, and then evaporate to dryness to obtain a white solid.
¹H NMR (500 MHz, Chloroform-*d*) δ 8.84-8.79 (m, 2H), 8.65-8.57 (m, 1H), 7.37-7.31 (m, 2H), 5.79-5.72, 5.55 (m, 1H), 4.57, 3.97-3.93 (m, 1H), 4.38-4.30 (m, 1H), 3.44-3.16 (m, 2H), 1.32-1.29, 1.18-1.16 (m, 3H).

### Examples 1-2: Preparation of precursor of KIDJI-004

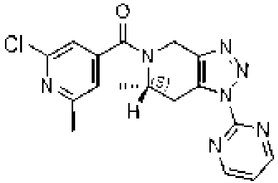

Following the method of Example 1-1, chloronicotinic acid was replaced with 2-chloro-6-methylisonicotinic acid to prepare (S)-(2-chloro-6-methylpyridin-4-yl)(6-methyl-1-(pyrimidin-2-yl)-1,4,6,7-tetrahydro-5*H*-[1,2,3]triazolo[4,5-c]pyridin-5-yl) methyl ketone (precursor of KIDJI-004).

### Examples 1-3:

Following the method of Example 1-1, (S)-2-methyl-4-oxoperidin-1-carboxylic acid tert-butyl ester was replaced with *N*-tert-butoxycarbonyl-4-piperidinone, and chloronicotinic acid was replaced with 3-n-butyltin benzoic acid to prepare (1-(pyrimidin-2-yl)-1,4,6,7-tetrahydro-5*H*-[1,2,3]triazolo[4,5-c]pyridin-5-yl)(4-tri-n-butyltin)phenyl) methyl ketone (precursor of KIDJI-007).

### Examples 1-4:

Following the method of Example 1-1, (S)-2-methyl-4-oxopiperidin-1-carboxylic acid tert-butyl ester was replaced with *N*-tert-butyloxycarbonyl-4-piperidinone, and chloronicotinic acid was replaced with 3-n-butyltin-4-methoxybenzoic acid to prepare (4-methoxy-3-(tri-n-butyltin)phenyl)(1-(pyrimidin-2-yl)-1,4,6,7-tetrahydro-5*H-*[1,2,3]triazolo[4,5-c]pyridin-5-yl) methyl ketone (precursor of KIDJI-008) .

### Examples 1-5:

Following the method of Example 1-1, (S)-2-methyl-4-oxoperidin-1-carboxylic acid tert-butyl ester was replaced with *N*-tert-butoxycarbonyl-4-piperidinone and chloronicotinic acid was replaced with 2-n-butyltin-4-nitrobenzoic acid to prepare (4-nitro-2-(tri-n-butyltin)phenyl)(1-(pyrimidin-2-yl)-1,4,6,7-tetrahydro-5*H-*[1,2,3]triazolo[4,5-c]pyridin-5-yl) methyl ketone (precursor of KIDJI-009).

### Examples 1-6:

Following a method similar to that in Examples 1-1, (S)-2-methyl-4-oxoperidin-1-carboxylic acid tert-butyl ester was replaced with *N*-tert-butoxycarbonyl-4-piperidinone and chloronicotinic acid was replaced with 2-fluoro-4-n-butyltin-benzoic acid to prepare (2-fluoro-4-(tri-n-butyltin)phenyl)(1-(pyrimidin-2-yl)-1,4,6,7-tetrahydro-5*H-*[1,2,3]triazolo[4,5-c]pyridin-5-yl) methyl ketone (precursor of KIDJI-010).

### Example 2: Label Synthesis

### Labeled Synthesis Example 2-1

### Synthesis of ¹⁸F-labeled (S)-(3-fluoro-2-(trifluoromethyl)pyridin-4-yl)(6-methyl-1-(pyrimidin-2-yl)-1,4,6,7-tetrahydro-5H-[1,2,3]triazolo[4,5-c]pyridin-5-yl) methyl ketone (¹⁸F-KIDJI-003)

Following the following pathway, (S)-(3-chloro-2-(trifluoromethyl)pyridin-4-yl)(6-methyl-1-(pyrimidin-2-yl)-1,4,6,7-tetrahydro-5H-[1,2,3]triazolo[4,5-c]pyridin-5-yl) methyl ketone (compound 7, precursor of KIDJI-003) was used as a precursor to prepare ¹⁸F labeled (S)-(3-fluoro-2-(trifluoromethyl)pyridin-4-yl)(6-methyl-1-(pyrimidin-2-yl)-1,4,6,7-tetrahydro-5*H*-[1,2,3]triazolo[4,5-c]pyridin-5-yl) methyl ketone (¹⁸F-KIDJI-003).

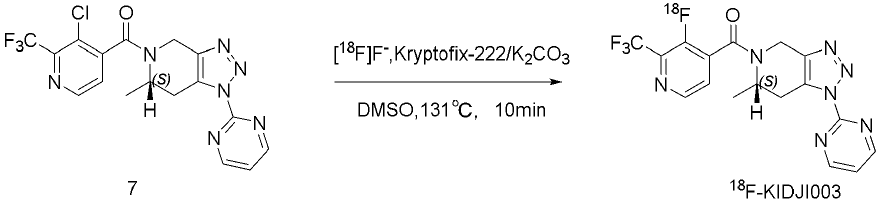

Weigh out 5 mg of amino polyether (kryptofix) 222 and 3 mg of K₂CO₃ and place them in a 1.5 mL EP tube. Add 100 µL of pure water and 400 µL of acetonitrile to prepare a kryptofix-222/K₂CO₃ solution. Take the kryptofix-222/K₂CO₃ solution and elute ¹⁸F- with a QMA column into a 3 mL pointed-bottom fluorination reaction tube and heat it at 135 °C to dry it under nitrogen for 3-5 minutes. Add 300 µL of anhydrous acetonitrile to the fluorination reaction tube, heat at 135 °C to dry, bubble with nitrogen for 2 minutes, and repeat three times to ensure thorough dehydration and drying.

Compound 7 (2 mg) was dissolved in anhydrous DMSO (200 µL), and 4 µL of KF (potassium fluoride) solution(1.5 mg/mL DMSO) was added. This mixture was then added to a reaction tube and stirred at 135 °C for 10 minutes. After the reaction was complete, the entire reaction solution was separated and purified by HPLC. HPLC was performed using a reverse-phase C18 column (Waters Atlantis T3 10×250mm, 5µm), the mobile phase was CH₃CN/H₂O (0.1% TFA) = 36:64 and the flow rate was 2.0 mL/min. Collect the fractions with a retention time of 37-38 minutes, transfer the resulting fraction of the labeled compound to a 20 mL rotary evaporator flask, concentrate under reduced pressure and dry in the rotary evaporator flask. Prepare ¹⁸F-KIDJI003 injection solution by adding physiological saline (containing 2.5% (V/V) Tween 80 and 2.5% (V/V) ascorbic acid solution). Radiochemical purity >95%. The molar activity is 61-87 GBq/ µmol.

¹H NMR (500 MHz, Chloroform-d) δ 8.95-8.91 (m, 2H), 8.68-8.64 (m, 1H), 7.60-7.56 (m, 1H), 7.49-7.44 (m,1H), 5.82, 5.65-5.62 (m,1H), 4.69, 4.19-4.16 (m,1H), 4.58-4.40 (m, 1H), 3.58-3.37 (m, 2H), 1.42-1.28 (m, 3H)

### Labeled Synthesis Example 2-2

### Synthesis of ¹⁸F-labeled (S)-(2-fluoro-6-methylpyridin-4-yl)(6-methyl-1-(pyrimidin-2-yl)-1,4,6,7-tetrahydro-5H-[1,2,3]triazolo[4,5-c]pyridin-5-yl) methyl ketone (¹⁸F-KIDJI-004)

Refer to the method of labeled synthesis Example 2-1, ¹⁸F-KIDJI-004 was prepared using (S)-(2-fluoro-6-methylpyridin-4-yl)(6-methyl-1-(pyrimidin-2-yl)-1,4,6,7-tetrahydro-5*H*-[1,2,3]triazolo[4,5-c]pyridin-5-yl) methyl ketone as a precursor. Radiochemical purity >95%. The molar activity is 58-90 GBq/µmol.

### Labeled Synthesis Examples 2-3:

### Synthesis of ¹³¹I labeled (4-iodophenyl)(1-(pyrimidin-2-yl)-1,4,6,7-tetrahydro-5H-[1,2,3]triazolo[4,5-c]pyridin-5-yl) methyl ketone (¹³¹I-KIDJI-007)

¹³¹I-KIDJI007 was prepared using (1-(pyrimidin-2-yl)-1,4,6,7-tetrahydro-5*H-*[1,2,3]triazolo[4,5-c]pyridin-5-yl)(4-tri-n-butyltin)phenyl)methyl ketone as a precursor, following the route described below.

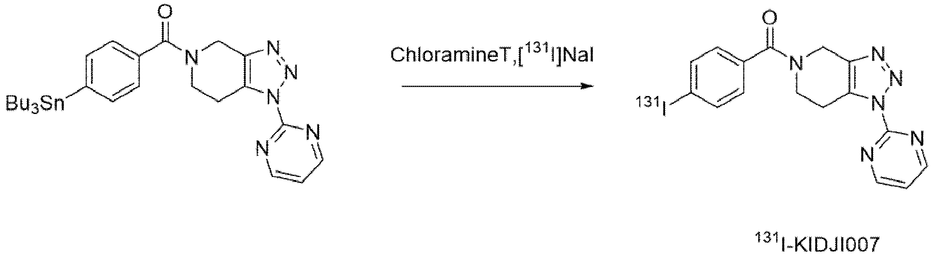

Place 65 µL of phosphate buffer (0.3 M, pH 5.5) in a 1.5 mL low-adsorption reaction tube, add 20 µL of the precursor (1 mg/mL methanol), add 5 µL of ¹³¹I-NaI (200 µCi, Xinke Pharmaceutical Co.), add 5 µL of chloramine T (0.4 mg/mL H₂O), vortex the reaction at room temperature for 3 min, and add 100 µL of Na₂S₂O₅ (2.0 mg/mL H₂O) to quench the reaction. Purification was performed using high performance liquid chromatography (column, CAPCELLPAK C18 UG120 (φ 6.0mm×150mm; Shiseido, Tokyo, Japan); mobile phase, CH₃CN/H₂O, 10/90~100/0, v/v; flow rate, 1.0 mL/min), and radioactive products were collected at the corresponding time points. Repeat the above process until enough radioactive drug(1.2-3mCi) was collected. Concentrate under reduced pressure until dry, then add physiological saline (containing 2.5% (V/V) Tween 80 and 2.5% (V/V) ascorbic acid solution) to prepare a radiolabeled product injection solution. The radiochemical purity is not less than 95%.

### Labeled Synthesis Examples 2-4:

### Synthesis of ¹³¹I-labeled (3-iodo-4-methoxyphenyl)(1-(pyrimidin-2-yl)-1,4,6,7-tetrahydro-5H-[1,2,3]triazolo[4,5-c]pyridin-5-yl) methyl ketone (¹³¹I-KIDJI-008)

Following the method described in Labeled Synthesis Example 2-3, ¹³¹I-KIDJI-008 was prepared using (4-methoxy-3-(tri-n-butyltin)phenyl)(1-(pyrimidin-2-yl)-1,4,6,7-tetrahydro-5*H*-[1,2,3]triazolo[4,5-c]pyridin-5-yl) methyl ketone as a precursor. Radiochemical purity >95%.

### Labeled Synthesis Examples 2-5:

### Synthesis of ¹³¹I-labeled (2-iodo-4-nitro)(1-(pyrimidin-2-yl)-1,4,6,7-tetrahydro-5H-[1,2,3]triazolo[4,5-c]pyridin-5-yl) methyl ketone (¹³¹I-KIDJI-009)

Following the method described in Labeled Synthesis Example 2-3, ¹³¹I-KIDJI-009 was prepared using (4-nitro-2-(tri-n-butyltin)phenyl)(1-(pyrimidin-2-yl)-1,4,6,7-tetrahydro-5*H*-[1,2,3]triazolo[4,5-c]pyridin-5-yl) methyl ketone as a precursor. Radiochemical purity >95%.

### Labeled Synthesis Examples 2-6:

### Synthesis of ¹³¹I-labeled (2-fluoro-4-iodophenyl)(1-(pyrimidin-2-yl)-1,4,6,7-tetrahydro-5H-[1,2,3]triazolo[4,5-c]pyridin-5-yl) methyl ketone (¹³¹I-KIDJI-010)

Following the method described in Labeled Synthesis Example 2-3, ¹³¹I-KIDJI-010 was prepared using (2-fluoro-4-(tri-n-butyltin)phenyl)(1-(pyrimidin-2-yl)-1,4,6,7-tetrahydro-5*H*-[1,2,3]triazolo[4,5-c]pyridin-5-yl) methyl ketone as the precursor. Radiochemical purity >95%.

### Example 3: Ex vivo autoradiography of ¹⁸F-KIDJI-003

The spontaneous type 2 diabetes mice model was developed using the db/db homozygous mice (BKS.Cg-Dock7m+/+LeprdbJ; JAX^{®} Mice Strain Code 607) discovered by the Jackson Laboratory in the United States. A defect in the leptin receptor gene on chromosome 4 causes db/db mice to exhibit overeating and obesity from four weeks of age, and as they grow older, they develop obvious hyperglycemia, hyperlipidemia, and insulin resistance. Their pathogenesis is very similar to that of subjects with type 2 diabetes. The control group mice were either normal mice (m/m) or heterozygous mice (m/db).

Mice were anesthetized with 1.5% (v/v) isoflurane and then injected with ¹⁸F-KIDJI-003 (15 MBq) via the tail vein. Sixty minutes later, the mice were euthanized by dislocation, and their kidneys were removed and rapidly frozen in dry ice. 20 µm frozen sections were prepared using a cryostat (Reward, RWD Minux FS800). Frozen sections were held in contact with the developing plate (FUJIFILM; BAS-IP SR 2025E) for 2 hours, and images were read and quantitatively analyzed using an autoradiography scanner (CR-35 Bio; Elysia-Raytest).

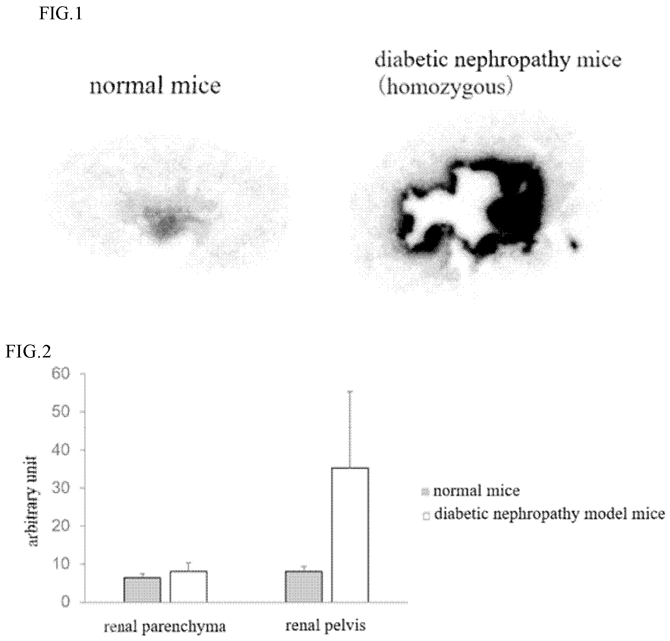

FIG.1 shows an ex vivo autoradiography. Compared with normal mice, ¹⁸F-KIDJI-003 showed significant accumulation in the renal pelvis of diabetic nephropathy mice. The results of quantitative analysis (FIG.2) showed that the radioactivity of ¹⁸F-KIDJI-003 in the renal pelvis of diabetic nephropathy mice was 4 times or more than that in the renal pelvis of normal mice. Data were obtained from the left and right kidneys of two normal mice and two diabetic nephropathy mice, with a total of four kidneys in each group.

### Example 4: in vivo imaging

In vivo imaging Example 4-1: PET in vivo imaging of ¹⁸F-KIDJI-004

PET scans were performed using an Inveon scanner (Siemens Medical Solutions Knoxville, TN, USA). Mice (m/m and db/db mice) were anesthetized with 1.5% (v/v) isoflurane and then fixed in the center of the FOV of a PET scanner. Immediately after intravenous injection of ¹⁸F-KIDJI-004 (15 MBq), 90 minutes of radiation signals were acquired in three-dimensional mode. The energy window is 350-750 keV. After the PET scan was completed, mice were intravenously injected with iodofol (35%) (Optiray 350; Guerbet) at a dose of 5 µL/g body weight, followed immediately by a 30-second CT scan (X-ray source: 70 kV/88 mA, FOV: 60 mm). Mice were anesthetized with 1.5% (v/v) isoflurane during PET and CT scans.

In vivo imaging Example 4-2: SPECT in vivo imaging of ¹³¹I-KIDJI-007

SPECT scans were performed using the nanoScan SPECT/CT scanner (Mediso Medical Imaging Systems, Hungary). Mice were anesthetized with 1.5%-2.0% (v/v) isoflurane, then placed on a preheated scanning mice bed, and injected with ¹³¹I-KIDJI-007 (11.1 MBq) via the tail vein. Immediately afterward, the radiation signal was acquired in three-dimensional mode every 30 minutes for up to 2 hours. The energy window is set to 360-700 keV. After completing the SPECT scan, mice were injected with iodofol (Optiray 350; Guerbet) via the tail vein at a dose of 5 µL/g body weight, followed immediately by a 30-second CT scan (X-ray source: 50 kV/980 µA, 480 projections; mice were anesthetized with 1.5% (v/v) isoflurane during both the SPECT and CT scans).

In vivo imaging Example 4-3: in vivo imaging of ¹³¹I-KIDJI-008

Following the method similar to that of in vivo imaging example 4-2, the in vivo imaging of ¹³¹I-KIDJI-008 was performed by replacing ¹³¹I-KIDJI-007 with ¹³¹I-KIDJI-008.

In vivo imaging Example 4-4: in vivo imaging of ¹³¹I-KIDJI-009

Following the method similar to that of in vivo imaging example 4-2, the in vivo imaging of ¹³¹I-KIDJI-009 was performed by replacing ¹³¹I-KIDJI-007 with ¹³¹I-KIDJI-009.

In vivo imaging Example 4-5: in vivo imaging of ¹³¹I-KIDJI-010

Following the method similar to that of in vivo imaging example 4-2, the in vivo imaging of ¹³¹I-KIDJI-010 was performed by replacing ¹³¹I-KIDJI-007 with ¹³¹I-KIDJI-010.

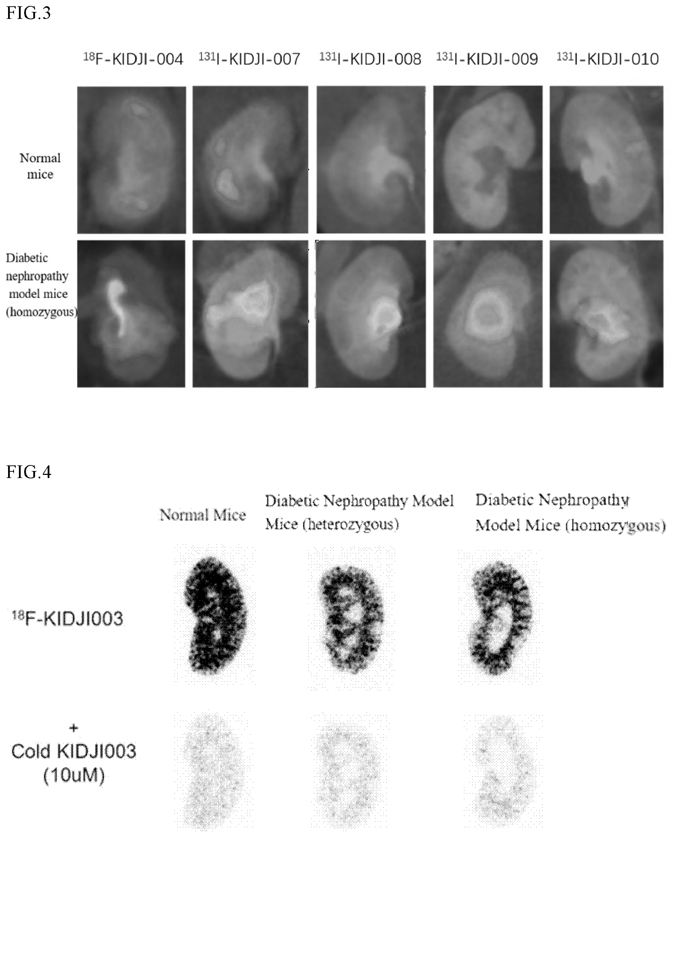

The results of in vivo imaging Examples 4 are shown in FIG. 3. Imaging results showed that ¹⁸F-KIDJI-004, ¹³¹I-KIDJI-007, ¹³¹I-KIDJI-008, ¹³¹I-KIDJI-009, and ¹³¹I-KIDJI-010 were significantly accumulated in the renal pelvis of the kidneys of db/db model mice, while no significant accumulation was observed in the kidneys of normal mice.

### Example 5: Ex vivo binding assay of kidney tissue sections in mice (In vitro autoradiography)

Freshly frozen kidneys from 6-month-old normal mice (m/m), heterozygous (m/db) and homozygous (db/db) diabetic nephropathy mice were prepared into frozen sections at a thickness of 20 µm. An incubation solution (final chemical concentration 5 nM) was prepared by adding ¹⁸F-KIDJI-003, which was labeled and synthesized in Example 2-1, into 50 mM Tris-HCl buffer (pH 7.4). Kidney sections were immersed in incubation solution with or without radiolabeled KIDJI-003 (10 µM), and after standing for 1 hour, they were washed twice for 2 minutes each time with 50 mM Tris-HCl buffer (pH 7.4). After being sealed in the developing plate for 1 hour, images were read and quantitatively analyzed using an autoradiography scanner (CR-35 Bio; Elysia-Raytest).

**Table 1**

| | TB | Non-SB | SB |
|---|---|---|---|
| normal mice | 72.49 | 13.83 | 58.66 |
| diabetic nephropathy mice (homozygous) | 54.87 | 14.13 | 40.74 |
| diabetic nephropathy mice (heterozygous) | 57.56 | 13.09 | 44.47 |

The upper part of FIG. 4 shows the total binding (TB) of ¹⁸F-KIDJI-003 on fresh frozen kidney sections from normal mice and diabetic nephropathy mice models. The lower part shows the in vitro autoradiography with unlabeled KIDJI-003, showing the non-specific binding (NSB) of ¹⁸F-KIDJI-003 on fresh frozen kidney sections from normal mice and diabetic nephropathy mice models. TB minus NSB equals specific binding (SB), which is a parameter index reflecting P2X7R expression. Table 1 shows the quantitative results of FIG.4. The addition of radio label-free KIDJI-003 significantly reduced the total binding of ¹⁸F-KIDJI-003, indicating a large amount of ¹⁸F-KIDJI-003-specific binding in mice kidneys. However, there was no difference in specific binding per unit area in the kidneys of normal mice and diabetic nephropathy mice models. This indicates that P2X7R expression was not increased in the diabetic nephropathy mice model. Therefore, the accumulation of ¹⁸F-KIDJI-003 in the renal pelvis of a diabetic nephropathy mice model is not due to the binding of ¹⁸F-KIDJI003 and P2X7R.

### Example 6 Immunostaining experiment of kidney tissue sections in mice

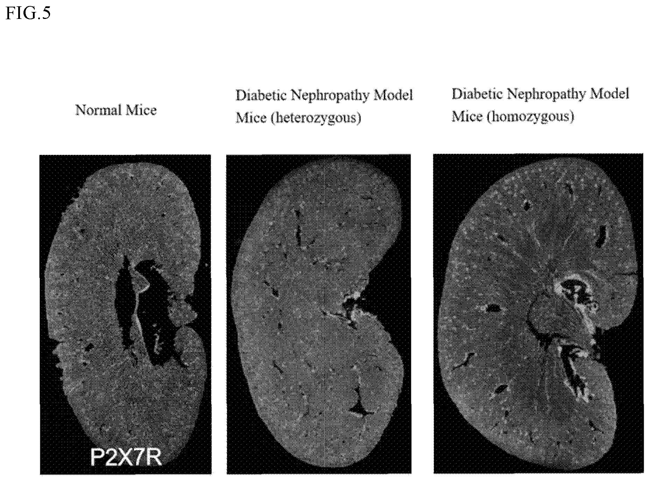

Six-month-old normal mice (m/m), heterozygous (m/db) and homozygous (db/db) mice were euthanized by cervical dislocation. The left and right kidneys were removed and immersed in 4% PFA/PBS fixative overnight to complete tissue fixation. After washing with PBS, the cells were sequentially immersed in 20% sucrose/PBS solution overnight and then in 30% sucrose/PBS solution overnight to complete the freeze protection. The kidneys were then prepared into 10 µm thick frozen sections using a cryostat (Reward). P2X7R staining was performed using an anti-P2X7R antibody (Cat#:APR-004, Alomone Labs Co.,). After being treated in an autoclave (citric acid buffer (0.01M sodium citrate: 0.01M citric acid = 5:1), 121°C, 5 minutes), the kidney sections were rinsed with running water for 5 minutes. TSA blocking buffer (Perkin Elmer Inc.,TSA Fluorescein System, NEL70000) was added to the kidney sections, and the sections were allowed to stand for 1 hour. Then, an incubation solution containing primary antibody (anti-P2X7R antibody, 1:1000) was added, and the sections were allowed to stand overnight. Discard the primary antibody and wash three times with PBS for 5 minutes each time. Add the incubation solution containing biotin-labeled secondary antibody and let it stand for 1 hour. Subsequently, the fluorescence signal was amplified using a TSA sensitization kit (TSA Fluorescein System, NEL70000; Perkin Elmer Inc.), and then mounted using VECTASHILD mounting medium (H-1000, Vector Laboratories Inc.) and observed under a microscope. The results are shown in FIG. 5. There was no significant difference in P2X7R expression in the kidneys of 6-month-old normal mice (left), heterozygous db/m (middle) diabetic nephropathy mice, and homozygous db/db (right) diabetic nephropathy mice. This result is consistent with the experimental results in FIG. 4, once again showing that P2X7R expression is not increased in the db/db homozygous diabetic nephropathy model mice. Therefore, this once again proves that the accumulation of ¹⁸F-KIDJI003 in the renal pelvis of a diabetic nephropathy mice model does not originate from the combination of ¹⁸F-KIDJI003 and P2X7R.

### Example 7: Diagnostic methods for diabetic nephropathy

In the diagnosis of DKD in subjects, similar to animal experiments, PET or SPECT imaging is performed after intravenous injection of the ¹⁸F-KIDJI003 radioactive molecular probe. Diabetic nephropathy is diagnosed based on the accumulation of radioactive molecular probes in the renal pelvis.

Although the present invention has been illustrated and described with reference to certain preferred embodiments, those skilled in the art should understand that the above description is a further detailed explanation of the present invention in conjunction with specific embodiments, and it should not be considered that the specific implementation of the present invention is limited to these descriptions. Without departing from the spirit and concept of this invention, those skilled in the art can make various changes in form and detail, including some simple deductions or substitutions, which are also part of this invention.

## Claims

1. A compound represented by general formula (I) or pharmaceutically acceptable salt, precursor, and solvate thereof, 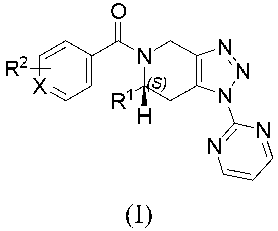 Wherein, X is carbon or nitrogen,
R¹ is selected from hydrogen or alkyl,
R² is one or more substituent on a benzene ring or pyridine ring, and R² is independently selected from one or more of hydrogen, halogen, hydroxyl, cyano, nitro, amino, C1-C6 alkyl, C3-C6 cycloalkyl, C2-C6 alkenyl, C2-C6 alkynyl, C1-C6 alkoxy, C3-C6 cycloalkoxy, C1-C6 alkylamino, C3-C6 cycloalkylamino, C1-C6 hydroxyalkyl, C3-C6 hydroxycycloalkyl, halo C1-C6 alkyl, halo C3-C6 cycloalkyl, halo C1-C6 alkoxy, halo C3-C6 cycloalkoxy, halo C1-C6 alkylamino, halo C3-C6 cycloalkylamino, C6-C8 aryl or C5-C8 heteroaryl, and in these substituents represented by R², at least one atom or the entire substituent is replaced by a radionuclide.

2. The compound, pharmaceutically acceptable salt, precursor, or solvate thereof as described in claim 1, wherein in general formula (I), R¹ is selected from hydrogen, C1-C6 alkyl, or C3-C6 cycloalkyl.

3. The compound, pharmaceutically acceptable salt, precursor, or solvate thereof as described in claim 1 or 2, wherein the radionuclide is selected from one or more of ¹⁸F, ¹¹C, ¹³¹I, ¹²³I, ¹²⁴I, or ¹²⁵I.

4. The compound, pharmaceutically acceptable salt, precursor, or solvate thereof as described in claim 1, wherein the compound is selected from: 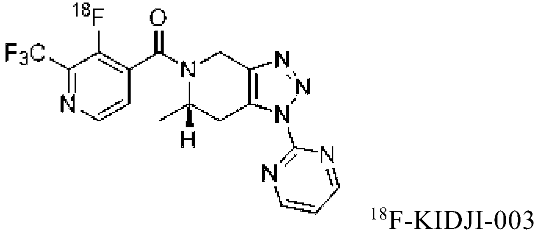
(S)-(3-fluoro-2-trifluoromethylpyridin-4-yl)(6-methyl-1-(pyrimidin-2-yl)-1,4,6,7-tetrahydro-5*H*-[1,2,3]triazolo[4,5-c]pyridin-5-yl) methyl ketone, 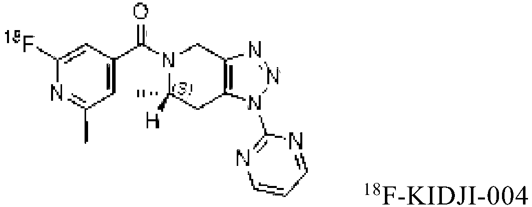
(S)-(2-fluoro-6-methylpyridin-4-yl)(6-methyl-1-(pyrimidin-2-yl)-1,4,6,7-tetrahydro-5*H*-[1,2,3]triazolo[4,5-c]pyridin-5-yl) methyl ketone, 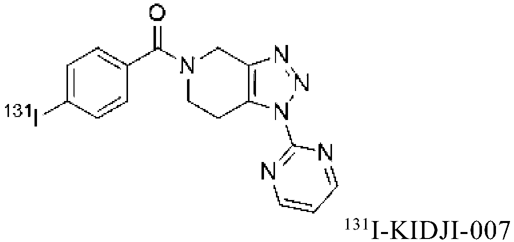
(4-iodophenyl)(1-(pyrimidin-2-yl)-1,4,6,7-tetrahydro-5*H*-[1,2,3]triazolo[4,5-c]pyridin-5-yl) methyl ketone, 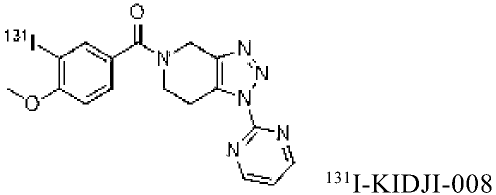
(3-iodo-4-methoxyphenyl)(1-(pyrimidin-2-yl)-1,4,6,7-tetrahydro-5H-[1,2,3]triazolo[4,5-c]pyridin-5-yl) methyl ketone, 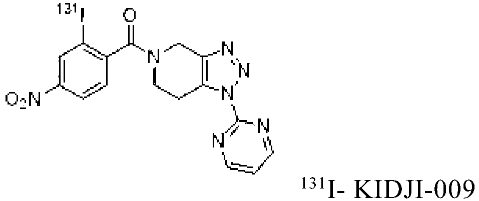
(2-iodo-4-nitro)(1-(pyrimidin-2-yl)-1,4,6,7-tetrahydro-5*H-*[1,2,3]triazolo[4,5-c]pyridin-5-yl) methyl ketone, 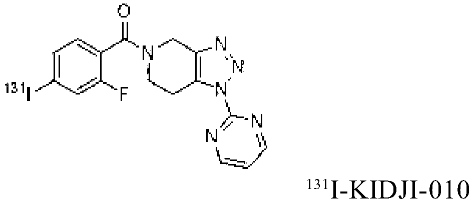
(2-fluoro-4-iodophenyl)(1-(pyrimidin-2-yl)-1,4,6,7-tetrahydro-5*H-*[1,2,3]triazolo[4,5-c]pyridin-5-yl) methyl ketone.

5. The compound, pharmaceutically acceptable salt, precursor, or solvate thereof as described in claim 1, wherein the precursor compound is selected from: 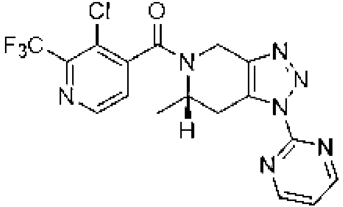
precursor of KIDJI-003 (S)-(3-chloro-2-trifluoromethylpyridin-4-yl)(6-methyl-1-(pyrimidin-2-yl)-1,4,6,7-tetrahydro-5*H*-[1,2,3]triazolo[4,5-c]pyridin-5-yl) methyl ketone, 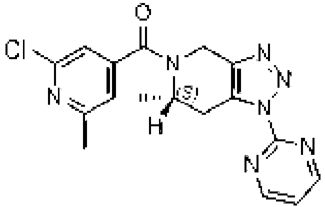
precursor of KIDJI-004 (S)-(2-chloro-6-methylpyridin-4-yl)(6-methyl-1-(pyrimidin-2-yl)-1,4,6,7-tetrahydro-5*H*-[1,2,3]triazolo[4,5-c]pyridin-5-yl) methyl ketone, 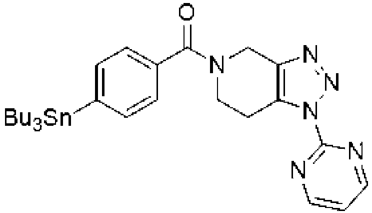 precursor of KIDJI-007 (1-(pyrimidin-2-yl)-1,4,6,7-tetrahydro-5*H*-[1,2,3]triazolo[4,5-c]pyridin-5-yl)(4-tri-n-butyltinphenyl) methyl ketone, 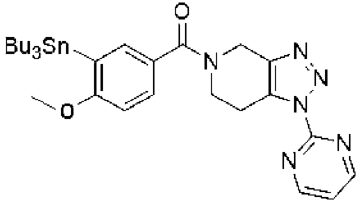
precursor of KIDJI-008 (4-methoxy-3-(tri-n-butyltin)phenyl)(1-(pyrimidin-2-yl)-1,4,6,7-tetrahydro-5*H-*[1,2,3]triazolo[4,5-c]pyridin-5-yl) methyl ketone, 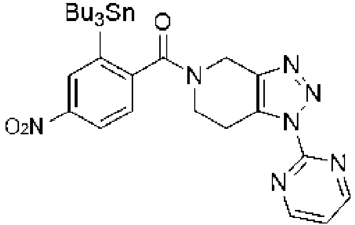
precursor of KIDJI-009 (4-nitro-2-(tri-n-butyltin)phenyl)(1-(pyrimidin-2-yl)-1,4,6,7-tetrahydro-5*H-*[1,2,3]triazolo[4,5-c]pyridin-5-yl) methyl ketone, 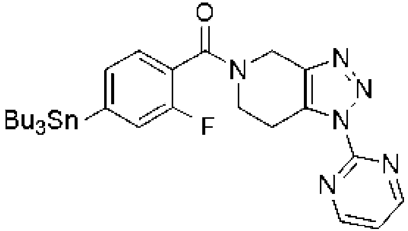
precursor of KIDJI-010 (2-fluoro-4-(tri-n-butyltin)phenyl)(1-(pyrimidin-2-yl)-1,4,6,7-tetrahydro-5*H-*[1,2,3]triazolo[4,5-c]pyridin-5-yl) methyl ketone.

6. The compound or pharmaceutically acceptable salt thereof as described in claim 1, wherein the pharmaceutically acceptable salt is an addition salt of an inorganic acid or an organic acid.

7. The compound or pharmaceutically acceptable salt thereof as described in claim 6, wherein the inorganic acid is selected from hydrochloric acid, hydrobromic acid, hydroiodic acid, sulfuric acid, phosphoric acid and nitric acid, and the organic acid is selected from acetic acid, tartaric acid, salicylic acid, methanesulfonic acid, succinic acid, citric acid, malic acid, lactic acid and fumaric acid.

8. The solvate of the compound as described in claim 1, wherein the solvate is a hydrate.

9. Use of the compound or pharmaceutically acceptable salt, precursor, or solvate thereof as described in any one of claims 1-8 in the preparation of a diagnostic medicament for diabetic nephropathy.

10. A radiodiagnostic kit for diagnosing diabetic nephropathy, the radiodiagnostic kit comprises a compound having the structure shown in general formula (I) or a pharmaceutically acceptable salt, precursor, or solvation thereof, 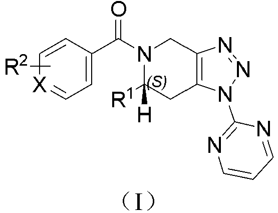 Wherein, X is carbon(C) or nitrogen(N),
R¹ is selected from hydrogen or alkyl,
R² is one or more substituent on a benzene ring or pyridine ring, and R² is independently selected from one or more of hydrogen, halogen, hydroxyl, cyano, nitro, amino, C1-C6 alkyl, C3-C6 cycloalkyl, C2-C6 alkenyl, C2-C6 alkynyl, C1-C6 alkoxy, C3-C6 cycloalkoxy, C1-C6 alkylamino, C3-C6 cycloalkylamino, C1-C6 hydroxyalkyl, C3-C6 hydroxycycloalkyl, halo C1-C6 alkyl, halo C3-C6 cycloalkyl, halo C1-C6 alkoxy, halo C3-C6 cycloalkoxy, halo C1-C6 alkylamino, halo C3-C6 cycloalkylamino, C6-C8 aryl or C5-C8 heteroaryl, and at least one atom of these substituents represented by R² is replaced by a radionuclide or the substituent as a whole is replaced by a radionuclide.

11. The radiodiagnostic kit as described in claim 9, wherein the pharmaceutically acceptable salt is an addition salt of an inorganic acid or an organic acid, and the solvate is a hydrate.

12. The radioactive diagnostic kit as described in claim 9, wherein the compound is selected from:

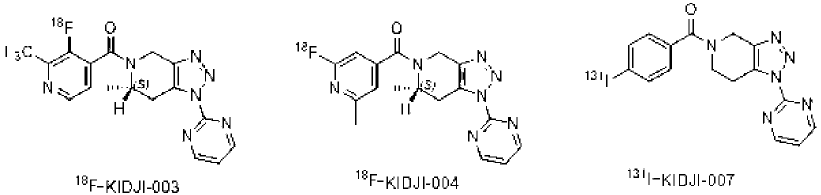

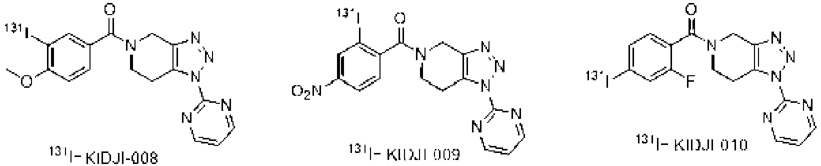

13. A method for diagnosing diabetic nephropathy including:
prepare a physiologically acceptable solution of the compound of any one of claims 1-4, 6-8, or a pharmaceutically acceptable salt or solvate thereof, or prepare a physiologically acceptable solution of the precursor of claim 5 after radionuclide labeling,
the solution was introduced into the subject's body,
image the subject's kidneys,
analyze the imaging results, and make a diagnosis based on the analysis results.

## Amended claims

### Amended claims under Art. 19.1 PCT

1. A compound represented by general formula (I) or pharmaceutically acceptable salt and solvate thereof, 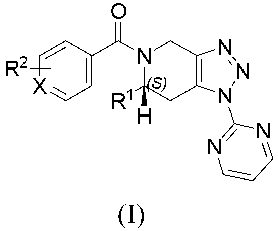 Wherein, X is carbon or nitrogen,
R¹ is selected from hydrogen or alkyl,
R² is one or more substituent on a benzene ring or pyridine ring, and R² is independently selected from one or more of hydrogen, halogen, hydroxyl, cyano, nitro, amino, C1-C6 alkyl, C3-C6 cycloalkyl, C2-C6 alkenyl, C2-C6 alkynyl, C1-C6 alkoxy, C3-C6 cycloalkoxy, C1-C6 alkylamino, C3-C6 cycloalkylamino, C1-C6 hydroxyalkyl, C3-C6 hydroxycycloalkyl, halo C1-C6 alkyl, halo C3-C6 cycloalkyl, halo C1-C6 alkoxy, halo C3-C6 cycloalkoxy, halo C1-C6 alkylamino, halo C3-C6 cycloalkylamino, C6-C8 aryl or C5-C8 heteroaryl, and in these substituents represented by R², at least one atom or the entire substituent is replaced by a radionuclide,
however, the compound of general formula (I) does not include

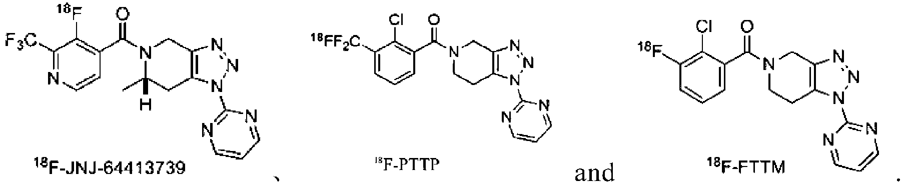

2. The compound, pharmaceutically acceptable salt, or solvate thereof as described in claim 1, wherein in general formula (I), R¹ is selected from hydrogen, C1-C6 alkyl, or C3-C6 cycloalkyl.

3. The compound, pharmaceutically acceptable salt, or solvate thereof as described in claim 1 or 2, wherein the radionuclide is selected from one or more of ¹⁸F, ¹¹C, ¹³¹I, ¹²³I, ¹²⁴I, or ¹²⁵I.

4. The compound, pharmaceutically acceptable salt, or solvate thereof as described in claim 1, wherein the compound is selected from: 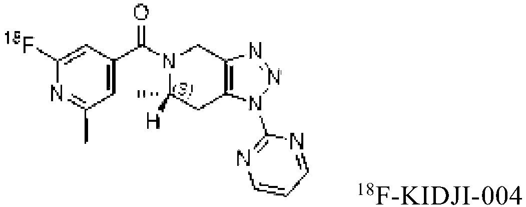
(S)-(2-fluoro-6-methylpyridin-4-yl)(6-methyl-1-(pyrimidin-2-yl)-1,4,6,7-tetrahydro-5*H*-[1,2,3]triazolo[4,5-c]pyridin-5-yl) methyl ketone, 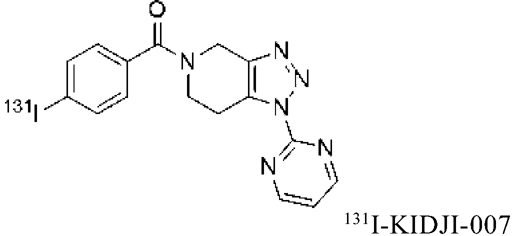
(4-iodophenyl)(1-(pyrimidin-2-yl)-1,4,6,7-tetrahydro-5*H*-[1,2,3]triazolo[4,5-c]pyridin-5-yl) methyl ketone, 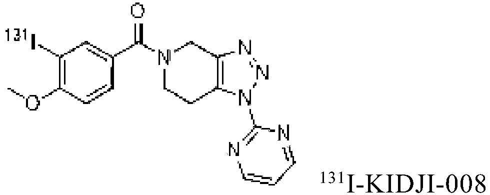
(3-iodo-4-methoxyphenyl)(1-(pyrimidin-2-yl)-1,4,6,7-tetrahydro-5*H-*[1,2,3]triazolo[4,5-c]pyridin-5-yl) methyl ketone, 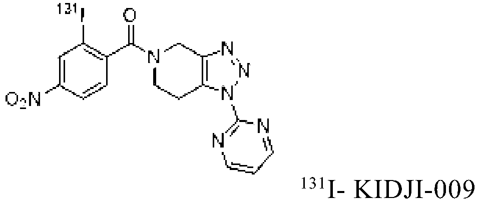
(2-iodo-4-nitro)(1-(pyrimidin-2-yl)-1,4,6,7-tetrahydro-5*H*-[1,2,3]triazolo[4,5-c]pyridin-5-yl) methyl ketone, 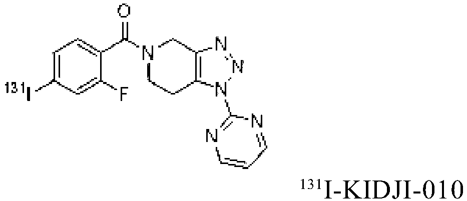
(2-fluoro-4-iodophenyl)(1-(pyrimidin-2-yl)-1,4,6,7-tetrahydro-5*H-*[1,2,3]triazolo[4,5-c]pyridin-5-yl) methyl ketone.

5. The compound or pharmaceutically acceptable salt thereof as described in claim 1, wherein the pharmaceutically acceptable salt is an addition salt of an inorganic acid or an organic acid.

6. The compound or pharmaceutically acceptable salt thereof as described in claim 5, wherein the inorganic acid is selected from hydrochloric acid, hydrobromic acid, hydroiodic acid, sulfuric acid, phosphoric acid and nitric acid, and the organic acid is selected from acetic acid, tartaric acid, salicylic acid, methanesulfonic acid, succinic acid, citric acid, malic acid, lactic acid and fumaric acid.

7. The solvate of the compound as described in claim 1, wherein the solvate is a hydrate.

8. Use of a compound shown in general formula (I) or a pharmaceutically acceptable salt or solvate thereof in the preparation of a diagnostic medicament for diabetic nephropathy, 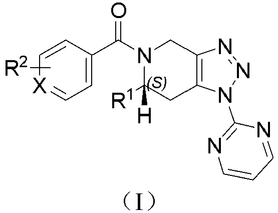 Wherein, X is carbon or nitrogen,
R¹ is selected from hydrogen or alkyl,
R² is one or more substituent on a benzene ring or pyridine ring, and R² is independently selected from one or more of hydrogen, halogen, hydroxyl, cyano, nitro, amino, C1-C6 alkyl, C3-C6 cycloalkyl, C2-C6 alkenyl, C2-C6 alkynyl, C1-C6 alkoxy, C3-C6 cycloalkoxy, C1-C6 alkylamino, C3-C6 cycloalkylamino, C1-C6 hydroxyalkyl, C3-C6 hydroxycycloalkyl, halo C1-C6 alkyl, halo C3-C6 cycloalkyl, halo C1-C6 alkoxy, halo C3-C6 cycloalkoxy, halo C1-C6 alkylamino, halo C3-C6 cycloalkylamino, C6-C8 aryl or C5-C8 heteroaryl, and at least one atom of these substituents represented by R² is replaced by a radionuclide or the substituent as a whole is replaced by a radionuclide.

9. Use as described in claim 8, wherein in general formula (I), R¹ is selected from hydrogen, C1-C6 alkyl, or C3-C6 cycloalkyl.

10. Use as described in claim 8, wherein the pharmaceutically acceptable salt is an addition salt of an inorganic acid or an organic acid, and/or, the solvate is a hydrate.

11. Use as described in claim 8, wherein the compound is selected from:

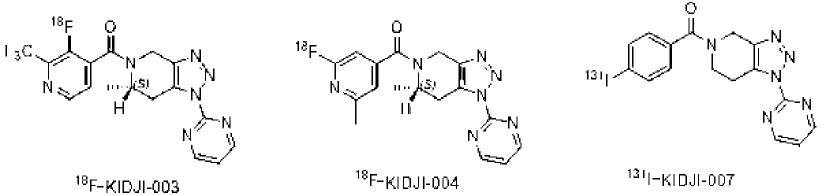

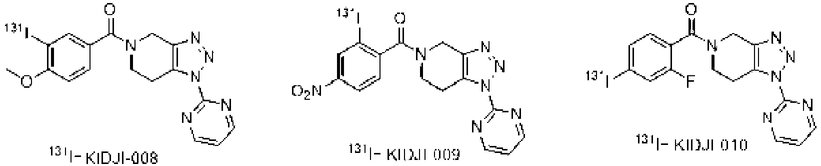

12. A radiodiagnostic kit for diagnosing diabetic nephropathy, the radiodiagnostic kit comprises a compound having the structure shown in general formula (I) or a pharmaceutically acceptable salt or solvation thereof, 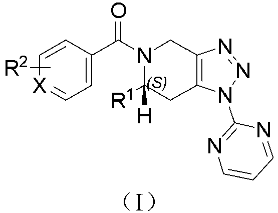 Wherein, X is carbon(C) or nitrogen(N),
R¹ is selected from hydrogen or alkyl,
R² is one or more substituent on a benzene ring or pyridine ring, and R² is independently selected from one or more of hydrogen, halogen, hydroxyl, cyano, nitro, amino, C1-C6 alkyl, C3-C6 cycloalkyl, C2-C6 alkenyl, C2-C6 alkynyl, C1-C6 alkoxy, C3-C6 cycloalkoxy, C1-C6 alkylamino, C3-C6 cycloalkylamino, C1-C6 hydroxyalkyl, C3-C6 hydroxycycloalkyl, halo C1-C6 alkyl, halo C3-C6 cycloalkyl, halo C1-C6 alkoxy, halo C3-C6 cycloalkoxy, halo C1-C6 alkylamino, halo C3-C6 cycloalkylamino, C6-C8 aryl or C5-C8 heteroaryl, and at least one atom of these substituents represented by R² is replaced by a radionuclide or the substituent as a whole is replaced by a radionuclide.

13. The radiodiagnostic kit as described in claim 12, wherein the pharmaceutically acceptable salt is an addition salt of an inorganic acid or an organic acid, and the solvate is a hydrate.

14. The radioactive diagnostic kit as described in claim 12, wherein the compound is selected from:

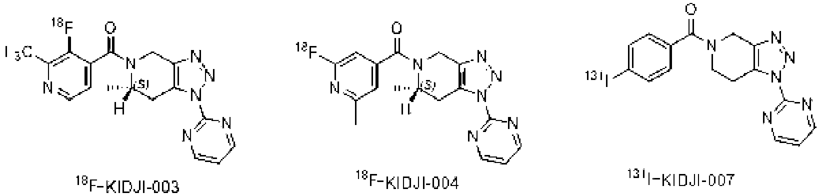

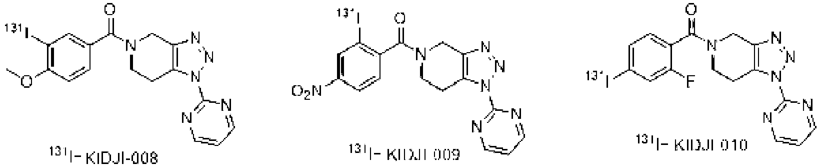

15. A method for diagnosing diabetic nephropathy including:
prepare a physiologically acceptable solution of the compound of any one of claims 1-7, or a pharmaceutically acceptable salt or solvate thereof,
the solution was introduced into the subject's body,
image the subject's kidneys,
analyze the imaging results, and make a diagnosis based on the analysis results.
